(19) Europäisches Patentamt
European Patent Office
Office européen des brevets

(11) Veröffentlichungsnummer: 0 088 039
B1

(12) **EUROPÄISCHE PATENTSCHRIFT**

(45) Veröffentlichungstag der Patentschrift: 12.10.88

(21) Anmeldenummer: 83710003.1

(22) Anmeldetag: 18.02.83

(51) Int. Cl.⁴: B 01 F 17/00, C 08 G 65/28, C 08 G 65/06, C 08 G 65/32, B 01 F 17/42

(54) **Neue Polyätherderivate, deren Verwendung als Emulgatoren sowie Emulsionen mit einem Gehalt an dem neuen Polyätherderivat.**

(30) Priorität: 03.03.82 DE 3207612

(43) Veröffentlichungstag der Anmeldung: 07.09.83 Patentblatt 83/36

(45) Bekanntmachung des Hinweises auf die Patenterteilung: 12.10.88 Patentblatt 88/41

(84) Benannte Vertragsstaaten: AT BE CH FR GB IT LI NL SE

(56) Entgegenhaltungen:
EP-A-0 024 472
US-A-4 207 421
US-A-4 252 540

**Die Akte enthält technische Angaben, die nach dem Eingang der Anmeldung eingereicht wurden und die nicht in dieser Patentschrift enthalten sind.**

(73) Patentinhaber: **Akzo Patente GmbH, Postfach 10 01 49 Kasinostrasse 19- 23, D-5600 Wuppertal- 1 (DE)**

(72) Erfinder: **Seibert, Karl, Dipl.- Ing., Am Hinzenbusch 62, D-5160 Düren- Niederau (DE)**

EP 0 088 039 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1988

**Beschreibung**

Gegenstand der Erfindung ist die Verwendung neue Polyätherderivate als Emulgatoren, hergestellt durch stufenweise Anlagerung eines langkettigen 1,2 Epoxides mit einer Kettenlänge von 10 bis 32 C-Atomen bei Temperaturen von 100 bis 200°C an Polyäthylenglykolmonoalkyläther, dessen mittleres Molekulargewicht 472 bis 2260 beträgt und dessen Alkylgruppe 1 bis 3 C-Atome enthält oder an einen Polyäthylenglykol-Polypropylenglykolmonoalkyläther, dessen mittleres Molekulargewicht 530 bis 2840 beträgt, dessen wiederkehrende Äthylenoxideinheiten und Propylenoxideinheiten je einen Block bilden, wobei der Polypropylenglykolblock ein mittleres Molekulargewicht von höchstens 580 aufweist, dessen Alkylgruppe das Ende des Polyäthylenglykolblocks bildet und 1 bis 3 C-Atome enthält, unter Verwendung von 0,1 bis 1 Gew.-% Alkalihydroxid, bezogen auf die Gesamtmenge der Ausgangsstoffe.

Diese Polyätherderivate entsprechen in ihrer Zusammensetzung der allgemeinen Formel

$$R_1{-}O(CH_2CH_2O)_m(CH_2\overset{CH_3}{\overset{|}{C}}HO)_n(CH_2{-}\overset{R_2}{\overset{|}{C}}HO)_p\cdot H$$

worin $R_1$ ein aliphatischer Kohlenwasserstoffrest mit 1 bis 3 C-Atomen
$R_2$ ein aliphatischer Kohlenwasserstoffrest mit 8 bis 30 C-Atomen
m = 10 - 50 (Mittelwert)
n = 0 - 10 (Mittelwert)
p = 1 - 10 (Mittelwert)
bedeutet.

Hergestellt werden die neuen Polyätherderivate analog den bekannten Umsetzungen von Alkoholen mit 1,2 Epoxiden, wie sie in Houben-Weyl, Methoden der organischen Chemie (1965) Band 14,2 Seiten 436 bis 450 beschrieben sind.

Emulgatoren sind bereits aus einer Vielzahl von Substanzklassen bekannt, beispielsweise Sorbitan, Fettsäureester und deren Äthoxylate, nichtionogene anionenaktive und kationenaktive Tenside, Phosphorsäurepartialestersalze und ähnliches. Dabei stehen Esterverbindungen im Vordergrund, die jedoch den Nachteil aufweisen, daß sie sehr hydrolyseempfindlich sind. Emulgatoren, die Aminstickstoff in gebundener Form enthalten, sind im allgemeinen toxisch und führen zu Hautreizungen.

Es sind aber auch schon Polyätherderivate beschrieben worden, die sich allerdings hinsichtlich ihres Aufbaus von den erfindungsgemäßen Polyätherderivaten unterscheiden, wie im folgenden dargelegt wird. So beschreibt US-A-4 207 421 biologisch abbaubare, alkalistabile, nichtionische oberflächenaktive Produkte einer allgemeinen Formel

$$R_1{-}O{-}(CH_2CH_2O)_a(CH_2\underset{R_2}{\underset{|}{C}}HO)_bH,$$

in der insbesondere die Reste $R_1$ ($C_{10-18}$-Alkyl) und $R_2$ ($C_{1-2}$Alkyl) sich von denjenigen der erfindungsgemäßen Polyätherderivate unterscheiden. Ferner beschreibt EP-A-0 024 472 Emulgatoren ("Grenzflächenspannung öliger Phasen in Wasser erniedrigende Verbindungen") der allgemeinen Formel

$$R_1[O(CH_2CH_2O)_x{-}(\underset{R'}{\underset{|}{C}}H{-}\underset{R''}{\underset{|}{C}}HO)_yH]_z,$$

in der sich insbesondere der Rest $R_1$ ($C_{8-22}$-Alkyl oder -Hydroxyalkyl) vom Rest $R_1$ der erfindungsgemäßen Produkte unterscheidet. Und schließlich beschreibt US-A-4 252 540 Stabilisatoren für Verbrennungsmischungen der allgemeinen Formel $R_1O(C_2H_4O)_1$-$(C_3H_6O)_m$-$(C_2H_4O)_n$-$R_2$, welche also anders als die erfindungsgemäßen Polyätherderivate aus Ethylenoxid-Propylenoxid-Ethylenoxid-Blöcken aufgebaut sind und insbesondere als Rest $R_2$ nur eine $C_{1-6}$ Alkylgruppe aufweisen, während $R_1$ ($C_{1-6}$ Alkyl) auch die erfindungsgemäßen Reste $R_1$ = $C_{1-3}$ Alkyl mit umfassen kann.

Die erfindungsgemäßen Polyätherderivate eignen sich besonders gut zu Emulgatoren, die im Gegensatz zu den meisten bekannten Emulgatoren sowohl in Öl-in-Wasser- als auch in Wasser-in-Öl-Emulsionen einsetzbar sind. Dabei sind sie sowohl für kosmetische als auch für technische Emulsionen hervorragend brauchbar. Im Vergleich zu Estern ist die besondere Stabilität gegenüber Verseifung hervorzuheben. Es werden kein Antioxidantien benötigt. Sie sind dermatologisch und toxikologisch unbedenklich.

Bevorzugte Polyätherderivate sind solche, bei denen die Kettenlänge des langkettigen Epoxides 12 bis 20 C-Atome ($R_2$ = 10 - 18) beträgt. Ebenfalls bevorzugt sind solche Polyätherderivate bei denen der Alkylrest ($R_1$) eine Methylgruppe ist.

Erfindungsgemäß werden die neuen Polyätherderivate als Emulgatoren in Emulsionen verwendet. Sie können je nach Molekülaufbau als wirksame Emulgatoren sowohl in Öl-in-Wasser-Emulsionen als auch in Wasser-in-Öl-Emulsionen verwendet werden und zwar erhält man dann Emulgatoren für Öl-in-Wasser-Emulsionen, wenn der Anteil an den Polyätherblöcken, die aus Polyäthylenoxideinheiten und gegebenenfalls aus Polypropylenoxideinheiten aufgebaut sind, mindestens 60 Gew.-% des gesamten Polyätherderivates ausmacht. Hervorragende Emulgatoren für Wasser-in-Öl-Emulsionen sind solche, bei denen der Anteil an den Polyätherblöcken, die aus Polyäthylenoxideinheiten und gegebenenfalls aus Polypropylenoxideinheiten aufgebaut sind, höchstens 50 Gew.-% des gesamten Polyätherderivates ausmachen.

Für kosmetische Emulsionen werden vor allem Wasser-in-Öl-Emulsionen bevorzugt, wozu sich die erfindungsgemäßen Emulgatoren ganz besonders eignen. Besonders hervorzuheben ist die spontane Emulgierwirkung der erfindungsgemäßen Emulgatoren und das glatte und glänzende Aussehen der damit hergestellten Cremes. Die Cremes sind nicht klebrig und sind auf der Haut gut verteilbar.

Die erfindungsgemäßen Emulgatoren sind farb- und geruchlos und gegen oxidative Einwirkungen beständig. Die hergestellten Emulsionen können von Personen mit empfindlicher Haut ohne Schwierigkeiten benutzt werden und hinterlassen auf der Haut ein angenehmes Gefühl. Die Parfümierung kann in engen Grenzen gehalten werden, da kein unangemehmer Eigengeruch der Emulgatoren überdeckt werden muß, was sich wiederum auf die Verträglichkeit bei Personen mit empfindlicher Haut vorteilhaft auswirkt.

Die Herstellung der erfindungsgemäßen Emulsionen erfolgt auf einfache und bekannte Weise durch Lösen der als Emulgator dienenden Polyätherderivate in der Öligen Phase bei erhöhter Temperatur von ca. 75°C, anschließende langsame Zugabe der auf ca. 75°C erwärmten Wasserphase unter Rühren der erhaltenen Emulsion. Inhaltsstoffe der herzustellenden, beispielsweise kosmetischen Emulsionen wie Hautfeuchtigkeitsregulatoren pflanzliche Wirkstoffauszüge, Vitamine, Hormone, Pigmente, Salze, Parfümöle, UV-Filterstoffe, Farbstoffe werden zweckmäßigerweise in der diese jeweils am besten aufnehmende Phase gelöst, beziehungsweise verteilt. Die erforderliche Menge an Emulgator beträgt 0,5 bis 10 Gew.-%, bezogen auf die gesamte Emulsion. Die einzuarbeitende Wassermenge kann 20 bis 80 Gew.-%, bezogen auf die gesamte Emulsion, betragen.

Als Ölphase der erfindungsgemäßen Emulsion kommen die üblicherweise verwendeten Produkte wie tierische und pflanzliche Öle und Fette, synthetische Ester von Fettsäuren mit aliphatischen Alkoholen, höhere Fettalkohole, Wachse, sogenannte mineralische Fette und Öle wie Paraffinöl, Vaseline®, Ceresin®, ferner Silikonöle und Silikonfette in Frage.

Die nachfolgenden Beispiele sollen die Erfindung näher erläutern.

**Beispiel 1**

In einem Dreihalskolben mit Rührer, absteigendem Kühler, Thermometer und Gaseinleitungsrohr wird das Alkoholäthoxylat unter Stickstoff-Atmosphäre aufgeschmolzen und mit 0,5 % 45 %-iger wäßriger Kaliumhydroxidlösung versetzt. In 0,5 Stunden wird unter Durchleiten von Stickstoff auf 60°C erwärmt, Wasserstrahlvakuum angelegt, in 0,5 Stunden auf 100°C erhitzt und während 0,5 Stunden bei dieser Temperatur im Vakuum gehalten. Anschließend wird mit Stickstoff entspannt, in 0,5 Stunden auf 180°C erhitzt und während 1 Stunde Epoxyalkan zugetropft. Nach einer Nachreaktionszeit von 2 Stunden ist die Reaktion beendet. Es wird auf 100°C abgekühlt und mit 90 %-iger Milchsäure neutralisiert.

Gemäß diesen Herstellungsbedingungen wurden Produkte hergestellt, deren Zusammensetzung und Eigenschaften in der Tabelle 1 zusammengestellt sind.

**Tabelle1**

| Produkt | $R_1$ | m | n | p | $R_2$ | mittleres Molekular-gewicht | Erstarrungs-punkt | Dichte | Aussehen |
|---|---|---|---|---|---|---|---|---|---|
| A | $CH_3$ | 22,39 | 0 | 7,35 | $C_{10}H_{21}$ | 1.750 | - | 0,9454 g/cm³ bei 50°C | gelbl. viskose Flüssigkeit |
| B | $CH_3$ | 16,7 | 0 | 1,18 | $C_{10}H_{21}$ | 1.050 | 29 - 32,0°C | 1,0220 g/cm³ bei 50°C | helle Paste |
| C | $CH_3$ | 22,39 | 0 | 1,43 | $C_{16}H_{33}$ | 1.350 | 38,5 - 40,5°C | 0,9850 g/cm³ bei 70°C | weißes Wachs |
| D | $C_3H_7$ | 22,39 | 2 | 7,35 | $C_{10}H_{21}$ | 1.850 | - | 0,967 g/cm³ bei 50°C | gelbl. viskose Flüssigkeit |

Während die Produkte A und D W/O-EMULGATOREN darstellen, repräsentieren die Produkte B und C O/W-EMULGATOREN.

**Beispiel 2**

Herstellung der Emulsionen.

Die in den nachfolgenden Beispielen aufgeführten W/O- als auch O/W-Emulsionen wurden hergestellt, indem die erfindungsgemäßen Emulgatoren zusammen mit den übrigen Komponenten der Fettphase auf 75°C erhitzt wurden. Getrennt dazu wurden die Komponenten der Wasserphase ebenfalls auf 75°C erhitzt und anschließend der heißen Fettphase unter anfänglich schnellem Rühren langsam zugesetzt. Nach Abkühlen der fertigen Emulsion wird erforderlichenfalls parfümiert. Entsprechend wurden jeweils die Emulsionen hergestellt, in denen vergleichsweise handelsübliche Emulgatoren eingesetzt wurden.

Von allen erhaltenen Emulsionen wurde die Lagerstabilität über einen Zeitraum von mindestens 3 Monaten durch Beobachtung möglicher Öl- bzw. Wasserabscheidungen bewertet. Die Wärmestabilität wurde bei einer Temperatur von 45°C, die Kältestabilität bei Temperaturen von -5°C sowie die Stabilität bei 20°C festgestellt.

Die Bewertung der Lagerstabilität erfolgte durch Benotung:

1 = sehr gut
2 = brauchbar
3 = unbrauchbar

**Beispiel 3**

Wasser-in-Öl-Emulsion

<u>Fettphase</u>

3,0 Teile Emulgator
3,0 Teile Lanolin
5,0 Teile Lanolinalkohol
3,0 Teile Hydroxystearinsäuretriglycerid
14,0 Teile Paraffinöl
0,2 Teile Konservierungsmittel

<u>Wasserphase</u>

5,0 Teile Sorbitol 70 %
0,5 Teile Magnesiumsulfat
0,2 Teile Borax
65,6 Teile Wasser
0,5 Teile Parfümöl

Die Emulsionen wurden hergestellt unter Verwendung der Emulgatoren

E I = Emulgator A

E II = Emulgator D

E III = Sorbitanmonooleat

| Eigenschaften | E I | E II | E III |
|---|---|---|---|
| Lagerstabilität | 1 | 1 | 2 |
| Wärmestabilität | 1 | 1 | 3 |
| Kältestabilität | 1 | 2 | 3 |

**Beispiel 4**

Fettphase
3,0 Teile Emulgator
3,0 Teile PEG-20/Dodecylglycol Copolymer
5,0 Teile Mikrowachs
17,0 Teile Paraffinöl
2,0 Teile Lanolinalkohol
Wasserphase
5,0 Teile Sorbitol 70 %
65,0 Teile Wasser

Die folgenden Emulgatoren wurden verwendet:

E III = Emulgator A

E IV = Polyglycerinisostearat

E V = Decaglyceroldecaoleat

E VI = Gemisch aus Oleylphosphorsäureestern

| Eigenschaften | E III | E IV | E V | E VI |
|---|---|---|---|---|
| Lagerstabilität | 1 | 1 | 1 | 1 |
| Wärmestabilität | 1 | 2 | 2 | 2 |
| Kältestabilität | 1 | 2 | 2 | 2 |

**Beispiel 5**

Fettphase
3,0 Teile Emulgator
5,0 Teile Absorptionsbase
5,0 Teile Bienenwachs
20,0 Teile Isopropylstearat
Wasserphase
5,0 Teile Sorbitol 70 %
62,0 Teile Wasser
Die folgenden Emulgatoren wurden verwendet:

E VII = Emulgator A

E VIII = Polyglycerinisostearat

| Eigenschaften | E VII | E VIII |
|---|---|---|
| Lagerstabilität | 1 | 1 |
| Wärmestabilität | 1 | 2 |
| Kältestabilität | 1 | 2 |

**Beispiel 6**

Fettphase
2,0 Teile Emulgator
5,0 Teile Isocetylstearat
5,0 Teile Glycerinmonostearat (90 %)
6,0 Teile Stearinsäure
0,2 Teile Konservierungsmittel
Wasserphase
3,0 Teile Glycerin
0,3 Teile Konservierungsmittel
77,2 Teile Wasser
0,3 Teile Parfümöl
Die folgenden Emulgatoren wurden verwendet:

E IX = Emulgator B

E X = Emulgator C

E XI = Polyoxyäthylenglycerinmonooleat

| Eigenschaften | E IX | E X | E XI |
|---|---|---|---|
| Lagerstabilität | 1 | 1 | 1 |
| Wärmestabilität | 1 | 3 | 3 |
| Kältestabilität | 1 | 3 | 3 |

**Beispiel 7**

Fettphase
3,0 Teile Emulgator
2,0 Teile acetyliertes Lanolin
1,6 Teile Glycerinmonostearat
2,0 Teile Stearinsäure
6,0 Teile Paraffinöl
2,0 Teile Cetylalkohol
0,2 Teile Konservierungsmittel
Wasserphase
5,0 Teile Sorbitol 70 %
1,5 Teile Magnesiumaluminiumsilikat
0,3 Teile Konservierungsmittel
Teile Wasser
0,2 Teile Parfümöl
Die folgenden Emulgatoren wurden verwendet:

E XII = Emulgator B

E XIII = Emulgator C

E XIV = Polyoxyäthylenmethylglucosidsesquistearatester

| Eigenschaften | E XII | E XIII | E XIV |
|---|---|---|---|
| Lagerstabilität | 1 | 1 | 3 |
| Wärmestabilität | 1 | 1 | 3 |
| Kältestabilität | 1 | 2 | 3 |

**Beispiel 8**

Öl-in-Wasser-Emulsion

Fettphase

3,0 Teile Emulgator
6,0 Teile Montanwachs
9,0 Teile Isopropylstearat
2,0 Teile Sesamöl
0,02 Teile Antioxidantien
0,2 Teile Konservierungsmittel

Wasserphase

0,3 Teile Triethanolamin
0,3 Teile Carboxyvinylpolymer
0,3 Teile Konservierungsmittel
78,68 Teile Wasser
0,2 Teile Parfümöl

Die folgenden Emulgatoren wurden verwendet:

E XV = Emulgator B

E XVI = Emulgator C

E XVII = Gemisch von Fettalkoholtetraglycolphosphorsäureester

| Eigenschaften | E XV | E XVI | E XVII |
|---|---|---|---|
| Lagerstabilität | 1 | 1 | 1 |
| Wärmestabilität | 1 | 1 | 3 |
| Kältestabilität | 1 | 1 | 2 |

**Patentansprüche**

1. Verwendung eines Polyätherderivates, als Emulgator in Emulsionen hergestellt durch stufenweise Anlagerung eines langkettigen 1,2 Epoxides mit einer Kettenlänge von 10 bis 32 C-Atomen bei Temperaturen von 100 bis 200°C an Polyäthylenglykolmonoalkyläther, dessen mittleres Molekulargewicht 472 bis 2260 beträgt und dessen Alkylgruppe 1 - 3 C-Atome enthält oder an einen Polyäthylenglykol-Polypropylenglykolmonoalkyläther, dessen mittleres Molekulargewicht 530 bis 2840 beträgt, dessen wiederkehrende Äthylenoxideinheiten und Propylenoxideinheiten je einen Block bilden, wobei der Polypropylenglykolblock ein mittleres Molekulargewicht von höchstens 580 aufweist, dessen Alkyläthergruppe das Ende des Polyäthylenglykolblocks bildet und 1 bis 3 C-Atome enthält, unter Verwendung von 0,1 bis 1 Gew.-% Alkalihydroxid, bezogen auf die Gesamtmenge der Ausgangsstoffe.

2. Verwendung nach Anspruch 1, dadurch gekennzeichnet, daß die Kettenlänge des langkettigen Epoxids im Mittel 12 bis 20 C-Atome beträgt.

3. Verwendung nach den Ansprüchen 1 bis 2, dadurch gekennzeichnet, daß die Alkylgruppe eine Methylgruppe ist.

4. Verwendung nach den Ansprüchen 1 bis 3 als Emulgator in Wasser in Öl-Emulsion.

5. Emulsion mit einem Gehalt an Polyätherderivat nach den Ansprüchen 1 bis 3 in einer Menge von 0,5 bis 10 Gew.-%, bezogen auf die gesamte Emulsion und üblicherweise in Emulsionen eingesetzten Fetten und/oder Ölen, Wachsen, Fettalkoholen, Fettsäuren, Mineralölen und weiteren üblichen Hilfsstoffen und Wasser.

## Claims

1. The use of a polyether derivative as emulsifier in emulsions prepared by stepwise addition of a long-chain 1,2-epoxide containing 10 to 32 carbon atoms at temperatures of 100 to 200°C onto polyethylene glycol monoalkyl ether of which the average molecular weight is from 472 to 2260 and of which the alkyl group contains 1 to 3 carbon atoms or onto a polyethylene glycol/polypropylene glycol monoalkyl ether, of which the average molecular weight is from 530 to 2840, of which the recurring ethylene oxide units and propylene oxide units each form a block, the polypropylene glycol block having an average molecular weight of at most 580, of which the alkyl ether group forms the end of the polyethylene glycol block and contains 1 to 3 carbon atoms, using 0.1 to 1 % by weight alkali hydroxide, based on the total quantity of starting materials.

2. The use claimed in claim 1, characterized in that the long-chain epoxide has a chain length of on average 12 to 20 carbon atoms.

3. The use claimed in claims 1 and 2, characterized in that the alkyl group is a methyl group.

4. The use claimed in claims 1 to 3 as an emulsifier in water-in-oil emulsions.

5. An emulsion containing a polyether derivative according to claims 1 to 3 in a quantity of 0.5 to 10 % by weight, based on the emulsion as a whole, and fats and/or oils, waxes, fatty alcohols, fatty acids and mineral oils typically used in emulsions and other standard auxiliaries and water.

## Revendications

1. Utilisation d'un dérivé polyéther comme émulsifiant dans des émulsions préparées par addition progressive d'un 1,2-époxyde à longue chaîne, avec une longueur de chaîne de 10 à 32 atomes de carbone, à des températures de 100 à 200°C, sur un éther monoalkylique de polyéthylèneglycol, dont la masse moléculaire moyenne est de 472 à 2260 et dont le groupe alkyle comporte de 1 à 3 atomes de carbone, ou sur un éther monoalkylique de polyéthylèneglycol-polypropylèneglycol, dont la masse moléculaire moyenne est de 530 à 2840, dont les motifs oxyéthylènes forment une séquence ainsi que les motifs oxypropylènes, la séquence polypropylèneglycol présentant une masse moléculaire moyenne de 580 au plus, et dont le groupe éther d'alkyle constitue la terminaison de la séquence polyéthylèneglycol et comporte de 1 à 3 atomes de carbone, avec utilisation de 0,1 à 1 % en poids d'hydroxyde alcalin, par rapport à la quantité totale des produits de départ.

2. Utilisation conforme à la revendication 1, caractérisée en ce que la longueur de chaîne de l'époxyde à longue chaîne est en moyenne de 12 à 20 atomes de carbone.

3. Utilisation conforme aux revendications 1 à 2, caractérisée en ce que le groupe alkyle est un groupe méthyle.

4. Utilisation conforme aux revendications 1 à 3, comme émulsifiant dans des émulsions eau-dans-huile.

5. Emulsion avec une teneur en dérivé polyéther selon les revendications 1 à 3, en une quantité de 0,5 à 10 % en poids par rapport à l'émulsion totale, et avec les graisses et/ou huiles, cires, alcools gras, acides gras, huiles minérales et autres adjuvants habituels incorporés habituellement dans les émulsions, et de l'eau.